# EUROPÄISCHE PATENTSCHRIFT

(11) **EP 3 044 575 B1**
(45) Veröffentlichungstag und Bekanntmachung des Hinweises auf die Patenterteilung: **14.12.2016**
(21) Anmeldenummer: 14758300.9
(22) Anmeldetag: 01.09.2014
(51) Int. Cl.: G01N 27/404, G01N 33/00

(54) **FLÜSSIGER ELEKTROLYT FÜR EINEN ELEKTROCHEMISCHEN GASSENSOR**
LIQUID ELECTROLYTE FOR AN ELECTROCHEMICAL GAS SENSOR
ÉLECTROLYTE LIQUIDE POUR UN CAPTEUR DE GAZ ÉLECTROCHIMIQUE

(30) Priorität: 09.09.2013 DE 102013014995
(43) Veröffentlichungstag der Anmeldung: 20.07.2016
(60) Teilanmeldung: 16002306.5
(73) Patentinhaber: Dräger Safety AG & Co. KGaA, 23560 Lübeck (DE)
(72) Erfinder: NAUBER, Andreas, 23617 Stockelsdorf (DE); SICK, Michael, 23669 Timmendorfer Strand (DE); STEINER, Gregor, 79822 Titisee-Neustadt (DE); MATTERN-FRÜHWALD, Marie-Isabell, 22941 Bargteheide (DE); CHRZAN, Rigobert, 23843 Bad Oldesloe (DE); SOMMER, Sabrina, 23558 Lübeck (DE); METT, Frank, 23556 Lübeck (DE); HENGSTENBERG, Andreas, 23858 Reinfeld (DE)
(74) Vertreter: Kettenbeil, Roxane Henriette
(86) Internationale Anmeldenummer: PCT/EP2014/002362
(87) Internationale Veröffentlichungsnummer: WO 2015/032480

(56) Entgegenhaltungen:
- EP-A1- 2 224 018
- WO-A1-2013/045561
- GB-A- 2 225 859
- XIAOBO JI ET AL: "Determination of ammonia based on the electrochemical oxidation of N,N'-diphenylenediamine in propylene carbonate", ANALYTICAL SCIENCES, Bd. 23, November 2007 (2007-11), Seiten 1317-1320, XP055154055,
- GIOVANELLI D ET AL: "Determination of ammonia based on the electro-oxidation of hydroquinone in dimethylformamide or in the room temperature ionic liquid, 1-ethyl-3-methylimidazolium bis(trifluoromethylsulfonyl)imide", TALANTA, Bd. 62, Nr. 5, April 2004 (2004-04), Seiten 904-911, XP055153888, ISSN: 0039-9140, DOI: 10.1016/j.talanta.2003.10.015
- LOPEZ DE MISHIMA B A ET AL: "Ammonia sensor based on propylene carbonate", SENSORS AND ACTUATORS B: CHEMICAL, Bd. 131, Nr. 1, 14. April 2008 (2008-04-14), Seiten 236-240, XP022602889, ISSN: 0925-4005, DOI: 10.1016/J.SNB.2007.11.012 [gefunden am 2007-11-21]

## Beschreibung

Die Erfindung betrifft einen flüssigen Elektrolyt für einen elektrochemischen Gassensor, insbesondere für einen elektrochemischen Gassensor zum Nachweis von NH₃ oder NH₃-haltigen Gasgemischen.

Elektrochemische Gassensoren, mit denen über einen begrenzten Zeitraum die Konzentration von gasförmigem Ammoniak (NH₃) detektierbar ist, sind allgemein bekannt. Solche Sensoren kommen üblicherweise in den verschiedensten technischen Bereichen zum Einsatz, von der chemischen Industrie über die Überwachung von Kühlanlagen bis hin zu landwirtschaftlichen Betrieben. Sie dienen insbesondere dazu kritische Konzentrationen des entzündlichen, bei Einatmung giftigen und ätzenden Ammoniak-Gases rechtzeitig zu erkennen und vor einer entsprechenden Gefahr zu warnen.

Eines der wesentlichen Bestandteile eines solchen elektrochemischen Sensors ist der in dem Sensor verwendete Elektrolyt. Der Elektrolyt steht dabei in leitendem Kontakt mit wenigstens einer Anode und einer Kathode. Tritt das nachzuweisende Gas in den elektrochemischen Sensor ein, so findet typischerweise zwischen dem Gas, dem Elektrolyten und dem Elektrodenmaterial eine Reaktion statt, die zu einem messbaren Stromfluss zwischen der Anode und der Kathode des Sensors führt.

So beschreibt EP 0 395 927 B1 eine elektrochemische Messzelle zur Bestimmung von Ammoniak oder Hydrazin in einer gasförmigen oder flüssigen Messprobe mit mindestens einer Messelektrode und einer Gegenelektrode, die in einer mit einem lösliche Elektrolyten gefüllten Elektrolytkammer aufgenommen sind, welche zur Messprobe hin durch eine permeable Membran abgeschlossen ist.

Auch EP 0 556 558 B1 sieht eine elektrochemische Messzelle zur Bestimmung von Ammoniak, Aminen, Hydrazin und Hyrdazinderivaten vor. Hier wird vorgeschlagen, als Leitelektrolyten in der Elektrolytlösung ein hygroskopisches Alkali- oder Erdalkalisalz zu verwenden. Dies soll ein Austrocknen des Elektrolyten verhindern und auf diese Weise eine möglichst langfristige Verwendbarkeit des Sensors ermöglichen.

Der Nachweise von Ammoniak (NH₃) bei derart gestalteten elektrochemischen Sensoren erfolgt mit Hilfe einer elektrochemischen Reaktion zwischen dem in den Sensor einströmenden Ammoniakgas, den Elektroden und dem Elektrolyten des Sensors. Im Zuge dieser Reaktion wird eintretendes Ammoniakgas an der Messelektrode oxidiert. Die dabei entstehenden Ammonium-Ionen werden anschließend an der Gegenelektrode wieder deprotoniert. In diesem Zusammenhang kann es sich aber zum Beispiel als problematisch erweisen, dass als Nebenprodukt dieser Reaktion weitere Stickstoffverbindungen gebildet werden können, die zu einer Blockierung (Vergiftung) der Elektrodenoberflächen führen kann.

Ausgehend davon ist es Aufgabe der vorliegenden Erfindung, diese und andere Nachteile des Standes der Technik zu überwinden.

Als Lösung sieht die Erfindung einen flüssigen Elektrolyten entsprechend Anspruch 1 vor. Ausgestaltungen sind Gegenständer abhängigen Ansprüche.

Ein Elektrolyt gemäß dem Oberbegriff des Anspruchs 1 ist aus ANALYTICAL SCIENCES, Bd. 23, November 2007, Seiten 1317-1320, XP055154055; GB 2 225 859 A sowie TALANTA, Bd. 62, Nr. 5, April 2004, Seiten 904-911, XP055153888, ISSN 0039-9140 bekannt.

Bei einem flüssigen Elektrolyt für einen elektrochemischen Gassensor, insbesondere für einen elektrochemischen Gassensor, der zum Nachweis von NH₃ oder NH₃-haltigen Gasgemischen geeignet ist, sieht die Erfindung vor, dass der Elektrolyt wenigstens ein Lösungsmittel, ein Leitsalz und einen organischen Mediator enthält, wobei das Leitsalz eine ionische Flüssigkeit, ein anorganisches Salz, ein organisches Salz oder ein Gemisch daraus ist, und wobei der organische Mediator eine Polyhydroxyverbindung ist, welche bei Oxidation ein chinoides System bildet oder ein Naphtalin-System bildet und der ausgewählt ist aus der Gruppe enthaltend substituierte ortho-Dihydroxybenzole, substituierte para-Dihydroxybenzole, Dihydroxynaphtalin, substituiertes Dihydroxynaphtalin, Anthrahydrochinon oder substituiertes Anthrahydrochinon.

Insbesondere für elektrochemische Gassensoren, bei welchen Elektroden aus Edelmetall oder Kohlenstoffnanoröhren verwendet werden, kann ein solcher Elektrolyt mit großem Vorteil eingesetzt werden, um die Dauerbegasungsfestigkeit eines solchen Sensors zu verbessern. Insbesondere das Risiko einer wie oben beschriebenen Vergiftung kann auf diese Weise deutlich minimiert werden.

Dabei ist es von besonderem Vorteil, wenn der Elektrolyt einen Puffer enthält, wobei der Puffer bevorzugt eine Verbindung entsprechend

Formel I R¹-(CR²R³)ₙ-SO₃H

mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(Hydroxyalkyl)alkyl. Beispielsweise können R² und R³ unabhängig voneinander ausgewählt sein aus H, NH und OH, wobei n = 2 ist und R¹ ausgewählt ist aus der Gruppe enthaltend Piperazinyl, substituiertes Piperazinyl, N-morpholino, Cylcoalkyl, tris-(Hydroxyalkyl)alkyl. Vorstellbar ist zum Beispiel auch, dass R² und R³ unabhängig voneinander ausgewählt sind aus H, NH und OH, wobei n = 2 ist und R¹ ausgewählt ist aus der Gruppe enthaltend N-morpholino und tris-(Hydroxyalkyl)alkyl). Beispielsweise ist es dabei ganz besonders von Vorteil, wenn n = 2 oder n = 3 ist, wobei alle R² und R³ unabhängig voneinander ausgewählt sind aus H, NH und OH, und wobei R¹ ausgewählt ist aus [4-(2-Hydroxyethyl)-1]-piperazinyl, (N-morpholino), N-Cyclohexyl, tris-(Hydroxymethyl)methyl. Ganz besonders bevorzugt ist der Puffer 3-(N-morpholino)-propansulfonsäure oder 3-(N-morpholino)ethansulfonsäure. So ist es zum Beispiel denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, und wobei der Elektrolyt außerdem einen Puffer enthält, insbesondere einen Puffer, der ausgewählt ist aus 3-(N-morpholino)-propansulfonsäure oder 3-(N-morpholino)-ethansulfonsäure.

Um zu verhindern, dass der Elektrolyt nach einer gewissen Zeit austrocknet - z.B. wenn der Sensor im Dauerbetrieb verwendet werden soll - ist es außerdem vorteilhaft, wenn der Elektrolyt als weitere Komponente eine Komponente zur Erniedrigung des Dampfdruckes enthält. Dabei kann die weitere Komponente bevorzugt ein Alkylenglykol oder Polyalkylenglykol sein, besonderes bevorzugt Propylenglykol, Ethylenglykol oder ein Gemisch aus Propylenglykol und Ethylenglykol ist. So ist es zum Beispiel denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist und wobei der Elektrolyt außerdem wenigstens ein Alkylenglykol enthält, insbesondere ein Alkylenglykol, das ausgewählt ist aus Propylenglykol, Ethylenglykol oder einem Gemisch aus Proylenglykol und Ethylenglykol.

Weiterhin ist es günstig, wenn das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus. Denkbar ist beispielsweise, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist und wobei das Lösungsmittel Wasser ist. Alternativ ist auch vorstellbar, dass der Elektrolyt ein Gemisch aus einem Lösungsmittel, einem Leitsalz und einen organischen Mediator ist, wobei das Leitsalz eine ionische Flüssigkeit, ein anorganisches Salz, ein organisches Salz oder ein Gemisch daraus ist und wobei das Lösungsmittel Alkylencarbonat, insbesondere Propylencarbonat, Ethylencarbonat oder ein Gemisch aus Propylencarbonat und Ethylencarbonat ist. Dabei ist es insbesondere auch vorstellbar, dass der Elektrolyt ein Gemisch aus einem Lösungsmittel, einem Leitsalz und einem organischen Mediator ist, wobei das Leitsalz eine ionische Flüssigkeit, ein anorganisches Salz, ein organisches Salz oder ein Gemisch daraus ist, wobei der Elektrolyt außerdem einen Puffer enthält, insbesondere einen Puffer, der ausgewählt ist aus 3-(N-morpholino)-propansulfonsäure oder 3-(N-morpholino)-ethansulfonsäure und wobei das Lösungsmittel Alkylencarbonat, insbesondere Propylencarbonat, Ethylencarbonat oder ein Gemisch aus Propylencarbonat und Ethylencarbonat ist. Außerdem ist es denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, wobei der Elektrolyt außerdem wenigstens ein Alkylenglykol enthält, insbesondere ein Alkylenglykol, das ausgewählt ist aus Propylenglykol, Ethylenglykol oder einem Gemisch aus Proylenglykol und Ethylenglykol, und wobei Lösungsmittel Alkylencarbonat, insbesondere Propylencarbonat, Ethylencarbonat oder ein Gemisch aus Propylencarbonat und Ethylencarbonat ist.

Bevorzugt ist das Anion des Leitsalzes ausgewählt aus der Gruppe enthaltend Halogenide, Carbonat, Sulfonat, Phosphat und/oder Phosphonat, bevorzugt ein Anion ausgewählt aus der Gruppe enthaltend Alkyl-sulfonat, Alkenyl-Sulfonat, Aryl-Sulfonat, Alkyl-phosphat, Alkenyl-Phosphat, Aryl-Phosphat, substituiertes Alkyl-sulfonat, substituiertes Alkenyl-sulfonat, substituiertes Aryl-sulfonat, substituiertes Alkyl-phosphat, substituiertes Alkenyl-phosphat, substituiertes Aryl-phosphat, halogeniertes Phosphat, halogeniertes Sulfonat halogeniertes Alkyl-sulfonat, halogeniertes Alkenyl-sulfonat, halogeniertes Aryl-sulfonat, halogeniertes Alkyl-phosphat, halogeniertes Alkenyl-phosphat, halogeniertes Aryl-phosphat, besonders bevorzugt ein Anion ausgewählt aus der Gruppe enthaltend Fluorophosphat, Alkylfluorophosphat, Aryl-sulfonat, ganz besonders bevorzugt aus der Gruppe enthaltend Perfluoralkylfluorophosphat, Toluolsulfonat.

Dabei ist es vorteilhaft, wenn das Leitsalz als Kationen Metallionen, Oniumionen oder ein Gemisch aus Metallionen und Oniumionen enthält. Zum Beispiel können die Metallionen ausgewählt sein aus Alkalimetallionen oder Erdalkalimetallionen, bevorzugt aus Li, K und/oder Na. Günstig ist es, wenn die Oniumionen ausgewählt sind aus Ammonium-, Phosphonium, Guanidiniumkationen und heterocyclischen Kationen, bevorzugt ausgewählt aus Alkyl-Ammonium- und heterocyclischen Kationen, besonders bevorzugt ausgewählt aus Alkyl-Ammonium, Imidazolium und/oder substituierten Imidazoliumionen, wobei die substituierten Imidazolium-Ionen bevorzugt eine Struktur entsprechend 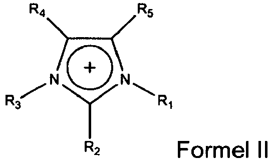 aufweisen, wobei R1, R2, R3, R4 und R5 unabhängig voneinander ausgewählt sein können aus -H, geradkettiges oder verzweigtes Alkyl mit 1 bis 20 C-Atomen, geradkettiges oder verzweigtes Alkenyl mit 2 bis 20 C-Atomen und einer oder mehreren Doppelbindungen, geradkettiges oder verzweigtes Aklinyl mit 2 bis 20 C-Atomen und einer oder mehreren Dreifachbindungen, gesättigtes, teilweise oder vollständig ungesättigtes Cycloalkyl mit 3-7 C-Atomen, das mit Alkylgruppen mit 1 bis 6 C-Atomen substituiert sein kann, gesättigtes teilweise oder vollständig ungesättigtes Heteroaryl, Heteroaryl-C1-C6-alkyl oder Aryl-C1-C6-alkyl, wobei besonders bevorzugt R2, R4 und R5 H sind und R1 und R3 jeweils unabhängig voneinander ein geradkettiges oder verzweigtes Alkyl mit 1 bis 20 C-Atomen.

Beispielsweise ist insbesondere vorstellbar, dass als Leitsalz Tetrabutlyammoniumtoluolsulfonat oder 1-Hexyl-3-methyl-imidazolium-tris(pentafluoroethyl)-trifluorophosphat verwendet wird. Alternativ ist auch vorstellbar, dass das Leitsalz zum Beispiel LiCl, KCl oder eine Mischung aus LiCl und KCl ist. So ist es insbesondere vorteilhaft, wenn der Elektrolyt ein Gemisch aus einem Lösungsmittel, einem Leitsalz und/oder einem organischen Mediator ist, wobei das Leitsalz ausgewählt ist aus LiCl, KCl, Alkylammonium-toluolsulfonat und ionischen Flüssigkeiten, mit einem Perfluoralkylfluorophosphat-Anion.

Weiterhin ist es günstig, wenn der organische Mediator ausgewählt ist aus der Gruppe enthaltend substituiertes 1,2- oder 1,4.Dihydroxybenzol, substituiertes Hydrochinon, substituiertes Naphtohydrochinon, besonders bevorzugt substituiertes Anthrahydrochinon, substituiertes Hydrochinon, substituiertes 1,2-Dihydroxybenzol. Dabei ist es besonders günstig, wenn die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt Sulfonsäure und/oder tert-Butyl.

In jedem Fall ist es besonders günstig, wenn der Elektrolyt als Lösungsmittel ein Gemisch aus Propylencarbonat und/oder Ethylencarbonat aufweist, als Leitsalz LiCl, KCl, Tetrabutylammoniumtoluolsulfonat und/oder 1-Hexyl-3-methyl-imidazolium-tris(pentafluoroethyl)-trifluorophosphat oder ein Gemisch aus zwei oder mehr dieser Komponenten aufweist und als organischen Mediator tert-butyl-Hydrochinon und/oder ein substituiertes Anthrachinon, bevorzugt Anthrachinon-2-Sulfonat aufweist.

Die Konzentration des organischen Mediators kann dabei zwischen 10-6 mol/ und 10-2 ml/l liegen. So kann der organische Mediator in einer Konzentration von 10⁻² mol/l oder weniger, bevorzugt von 10⁻³ mol/l oder weniger, besonders bevorzugt von 5*10⁻⁴ mol/l oder weniger, ganz besonders bevorzugt von 2*10⁻⁴ mol/l oder weniger, ganz besonders bevorzugt von 10⁻⁴ mol/l oder weniger in dem Elektrolyt enthalten sein. Denkbar ist auch, dass der organische Mediator in einer Konzentration von 10⁻⁶ mol/l oder mehr, bevorzugt von 10⁻⁵ mol/l oder mehr, besonders bevorzugt von 5*10⁻⁵ mol/l oder mehr, ganz besonders bevorzugt von 8*10⁻⁵ mol/l oder mehr, ganz besonders bevorzugt von 10⁻⁴ mol/l oder mehr in dem Elektrolyt enthalten ist. Insbesondere ist es auch denkbar, dass der organische Mediator in einer Konzentration von 10⁻⁵ mol/l bis 10⁻³ mol/l, bevorzugt 5*10⁻⁵ mol/l bis 5*10⁻⁴ mol/l, besonders bevorzugt 8*10⁻⁵ mol/l bis 2*10⁻⁴ mol/l, ganz besonders bevorzugt 10⁻⁴ mol/l vorhanden ist.

Ein erfindungsgemäßer Elektrolyt ist besonders bevorzugt erhältlich mit Hilfe eines Verfahrens, welches die folgenden Schritte umfasst:
a. Vorlegen des Lösungsmittels in einem Reaktionsgefäß
b. Zugabe des Puffers
c. Zugabe des organischen Mediators
d. Erhitzen des Gemisches unter Rühren für ca. 15 Minuten auf 150 °C
e. Rühren für etwa eine Stunde ohne weitere Hitzezufuhr bis alle Feststoffe gelöst sind
f. Abkühlen auf Raumtemperatur
g. Zugabe des Leitsalzes

Weitere Details und Einzelheiten ergeben sich aus den nachfolgend beschriebenen Figuren und Ausführungsbeispielen. Dabei zeigt
- Fig. 1: einen schematischen Aufbau eines elektrochemischen Gassensors mit welchem der erfindungsgemäße Elektrolyt zum Nachweis von Ammoniak verwendbar ist.
- Fig. 2: einen schematischen Ablauf einer Nachweisreaktion für NH₃ in einem elektrochemischen Gassensor, welcher einen erfindungsgemäßen Elektrolyten enthält.

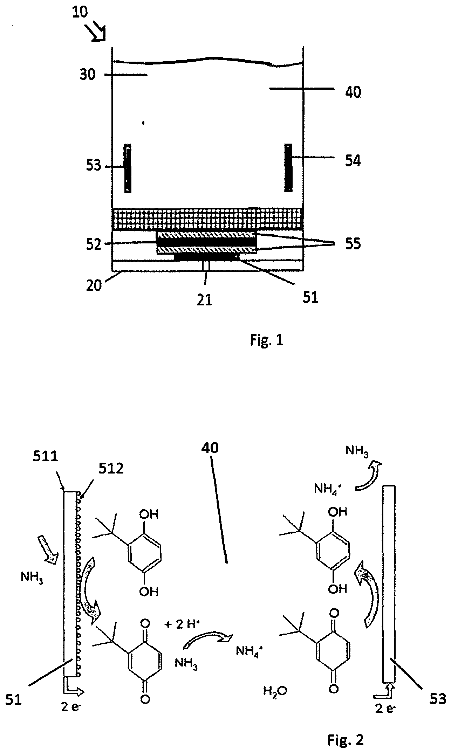

Man erkennt in Fig. 1, einen elektrochemischen Gassensor 10, welcher ein Gehäuse 20 mit einem Elektrolytreservoir 30 aufweist. In dem Gehäuse 20 ist ein Gaseinlass 21 und ein Gasauslass 22 ausgebildet. Innerhalb des Gehäuses 20 ist eine Arbeitselektrode 51 derart angeordnet, das sie in Kontakt mit Gas steht, das durch den Gaseinlass 21 in das Gehäuse 20 einströmt. Die Arbeitselektrode 51 ist mit Hilfe einer Glasfasermembran 55 von einer Abfangelektrode 52 getrennt. Die Abfangelektrode 52 ist ihrerseits mit einer Glasfasermembran 55 von dem Elektrolytreservoir 30 getrennt. Innerhalb des Elektrolytreservoirs 30 sind weiterhin eine Gegenelektrode 53 und eine Referenzelektrode 54 angeordnet.

In dem Elektrolytreservoir 30 ist der erfindungsgemäße Elektrolyt 40 vorhanden. Dabei sind die Glasfasermembranen 55 mit dem Elektrolyten durchtränkbar. Auf diese Weise kann der Elektrolyt 40 sowohl zur Arbeitselektrode 51 als auch zur Abfangelektrode 52 gelangen, so dass dort jeweils eine chemische Reaktion entsprechend dem in Fig. 2 dargestellten Schema zwischen einströmendem NH₃, dem Material der Arbeits- bzw. Abfangelektrode 51, 52 und dem Elektrolyten 40 stattfinden kann.

Dabei reagiert in den Gassensor 10 einströmendes NH3 an der Oberfläche der Arbeitselektrode 51 mit dem Elektrolyten. Bevorzugt besteht die Arbeitselektrode 51 dabei z.B. aus einer PTFE-Membran mit einer Kohlenstoff-Nanoröhren-Beschichtung. Die Gegenelektode 53 besteht bevorzugt aus einem Edelmetall. Der Elektrolyt 40 ist in diesem Beispiel eine Zusammensetzung aus Propylencarbonat und/oder Ethylencarbonat als Lösungsmittel, 1-Hexyl-3-methyl-imidazolium-tris(pentafluoroethyl)-trifluorophosphat als Leitsalz und tert-butyl-1, 2-Dihydroxybenzol als organischem Mediator. Der Elektrolyt enthält weiterhin bevorzugt einen Puffer, nämlich 3-(N-morpholino)propansulfonsäure. Wie in Fig. 2 erkennbar, wird das tert-Butyl-1,2-Dihydroxybenzol an der Arbeitselektode 51 zu tert-butyl-Chinon oxidiert. Die dabei freigesetzten Protonen reagieren mit dem in den Gassensor 10 einströmenden NH3 zu Ammoniumionen. Die Ammoniumionen gelangen zur Gegenelektrode 53, wo die Rückreaktion des zuvor gebildeten tert-butyl-Chinon zu 1,2-Dihydroxybenzol stattfindet. Dabei wird aus den Ammoniumionen wiederum NH₃ freigesetzt, das durch den Gasauslass 22 entweichen kann. Im Zuge dieses Reaktionsablaufes stabilisiert der eingesetzte Puffer den pH-Wert des Elektrolyten, der zwischen der Arbeits- und der Gegenelektrode 51, 53 im Elektrolytreservoir 30 vorliegt.

Ausführungsbeispiel für die Herstellung eines erfindungsgemäßen Elektrolyten:
In einem Reaktionsgefäß wird als Lösungsmittel Polycarbonat vorgelegte. Zu dem Polycarbonat wird 0,4 Gew.% Puffer, bevorzugt 3-(N-morpholinop)propansulfonsäure zugegeben. Im nächsten Schritt werden 6,9 Gew.% des organischen Mediators, bevorzugt tert-butyl-1,2-Dihydroxybenzol zugegeben. Die Mischung wird unter Rühren innerhalb von 15 Minuten erhitzt, wobei eine maximale Temperatur von 150°C nicht überschritten wird. Im Anschluss wurde die Mischung für eine Stunde ohne weitere Hitzezufuhr weiter gerührt bis alle Feststoffe gelöst waren. Die erhaltene Lösung hat eine klare, leicht gelbliche Farbe.

Die so erhaltene Lösung wird stehen gelassen, bis sie auf Raumtemperatur abgekühlt ist. Danach werden 2,7 Gew.% des Leitsalzes, bevorzugt Hmim-FAP (3-Hexyl-3-methyl-imidazolium-tris(pentafluoroethyl)-trifluorphosphat), zugegeben und das Gemisch wird kurz, für etwa 1 Minute, umgerührt.

**Bezugszeichenliste**

| | | | |
|---|---|---|---|
| 10 | Gassensor | 40 | Elektrolyt |
| | | | |
| 20 | Gehäuse | 51 | Arbeitselektrode |
| 21 | Gaseinlass | 52 | Abfangelektrode |
| 22 | Gasauslass | 53 | Gegenelektrode |
| | | 54 | Referenzelektrode |
| 30 | Elektrolytreservoir | 55 | Glasfasermembran |

## Patentansprüche

1. Flüssiger Elektrolyt für einen elektrochemischen Gassensor, insbesondere für einen elektrochemischen Gassensor, der zum Nachweis von NH₃ oder NH₃-haltigen Gasgemischen geeignet ist, wobei der Elektrolyt wenigstens ein Lösungsmittel, ein Leitsalz und einen organischen Mediator enthält, wobei das Leitsalz eine ionische Flüssigkeit, ein anorganisches Salz, ein organisches Salz oder ein Gemisch daraus ist **dadurch gekennzeichnet, dass** der organische Mediator eine Polyhydroxyverbindung ist, welche bei Oxidation ein chinoides System oder ein Naphthalin-System bildet wobei der organische Mediator ausgewählt ist aus der Gruppe enthaltend substituierte ortho-Dihydroxybenzole, substituierte para-Dihydroxybenzole, Dihydroxynaphtalin, substituiertes Dihydroxynaphtalin, Anthrahydrochinon, substituiertes Anthrahydrochinon.

2. Elektrolyt entsprechend Anspruch 1, **dadurch gekennzeichnet, dass** der Elektrolyt einen Puffer enthält, wobei der Puffer bevorzugt eine Verbindung entsprechend
Formel I R¹-(CR²R³)ₙ-SO₃H
mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(Hydroxyalkyl)alkyl.

3. Elektrolyt entsprechend Anspruch 2, **dadurch gekennzeichnet, dass** n = 2 oder n = 3 ist, wobei alle R² und R³ unabhängig voneinander ausgewählt sind aus H, NH und OH, und wobei R1 ausgewählt ist aus [4-(2-Hydroxyethyl)-1]-piperazinyl, (N-morpholino), N-Cyclohexyl, tris-(Hydroxymethyl)methyl, wobei der Puffer bevorzugt 3-(N-morpholino)propansulfonsäure oder 3-(N-morpholino)ethansulfonsäure ist.

4. Elektrolyt entsprechend einem der Ansprüche 1 bis 3, **dadurch gekennzeichnet, dass** der Elektrolyt als weitere Komponente eine Komponente zur Erniedrigung des Dampfdruckes enthält, wobei die weitere Komponente bevorzugt ein Alkylenglykol oder Polyalkylenglykol ist, besonderes bevorzugt Propylenglykol, Ethylenglykol oder ein Gemisch aus Propylenglykol und Ethylenglykol ist.

5. Elektrolyt entsprechend wenigstens einem der Ansprüche 1 bis 4, **dadurch gekennzeichnet, dass** das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus.

6. Elektrolyt entsprechend wenigstens einem der Ansprüche 1 oder 5, **dadurch gekennzeichnet, dass** das Anion des Leitsalzes ausgewählt ist aus der Gruppe enthaltend Halogenide, Carbonat, Sulfonat, Phosphat und/oder Phosphonat,
bevorzugt ein Anion ausgewählt aus der Gruppe enthaltend Alkyl-sulfonat, Alkenyl-Sulfonat, Aryl-Sulfonat, Alkyl-phosphat, Alkenyl-Phosphat, Aryl-Phosphat, substituiertes Alkyl-sulfonat, substituiertes Alkenyl-sulfonat, substituiertes Aryl-sulfonat, substituiertes Alkyl-phosphat, substituiertes Alkenyl-phosphat, substituiertes Aryl-phosphat, halogeniertes Phosphat, halogeniertes Sulfonat halogeniertes Alkyl-sulfonat, halogeniertes Alkenyl-sulfonat, halogeniertes Aryl-sulfonat, halogeniertes Alkyl-phosphat, halogeniertes Alkenyl-phosphat, halogeniertes Aryl-phosphat,
besonders bevorzugt ein Anion ausgewählt aus der Gruppe enthaltend Fluorophosphat, Alkylfluorophosphat, Aryl-sulfonat,
ganz besonders bevorzugt aus der Gruppe enthaltend Perfluoralkylfluorophosphat, Toluolsulfonat.

7. Elektrolyt entsprechend wenigstens einem der Ansprüche 1 bis 6, **dadurch gekennzeichnet, dass** das Leitsalz als Kationen Metallionen, Oniumionen oder ein Gemisch aus Metallionen und Oniumionen enthält.

8. Elektrolyt entsprechend Anspruch 7, **dadurch gekennzeichnet, dass** die Metallionen ausgewählt sind aus Alkalimetallionen oder Erdalkalimetallionen, bevorzugt aus Li, K und/oder Na.

9. Elektrolyt entsprechend einem der Ansprüche 7 oder 8, **dadurch gekennzeichnet, dass** die Oniumionen ausgewählt sind aus Ammonium-, Phosphonium, Guanidiniumkationen und heterocyclischen Kationen,
bevorzugt ausgewählt aus Alkyl-Ammonium- und heterocyclischen Kationen, besonders bevorzugt ausgewählt aus Alkyl-Ammonium, Imidazolium und/oder substituierten Imidazoliumionen, wobei die substituierten Imidazolium-Ionen bevorzugt eine Struktur entsprechend 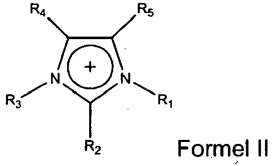 aufweisen, wobei R1, R2, R3, R4 und R5 unabhängig voneinander ausgewählt sein können aus -H, geradkettiges oder verzweigtes Alkyl mit 1 bis 20 C-Atomen, geradkettiges oder verzweigtes Alkenyl mit 2 bis 20 C-Atomen und einer oder mehreren Doppelbindungen, geradkettiges oder verzweigtes Alkinyl mit 2 bis 20 C-Atomen und einer oder mehreren Dreifachbindungen, gesättigtes, teilweise oder vollständig ungesättigtes Cycloalkyl mit 3-7 C-Atomen, das mit Alkylgruppen mit 1 bis 6 C-Atomen substituiert sein kann, gesättigtes teilweise oder vollständig ungesättigtes Heteroaryl, Heteroaryl-C1-C6-alkyl oder Aryl-C1-C6-alkyl, wobei besonders bevorzugt R2, R4 und R5 H sind und R1 und R3 jeweils unabhängig voneinander ein geradkettiges oder verzweigtes Alkyl mit 1 bis 20 C-Atomen.

10. Elektrolyt entsprechend einem der vorhergehenden Ansprüche, **dadurch gekennzeichnet,**
**dass** der organische Mediator ausgewählt ist aus der Gruppe enthaltend substituiertes 1,2- oder 1,4-Dihydroxybenzol, substituiertes Hydrochinon, substituiertes Naphtohydrochinon,
besonders bevorzugt substituiertes Anthrahydrochinon, substituiertes Hydrochinon, substituiertes 1,2-Dihydroxybenzol.

11. Elektrolyt entsprechend Anspruch 10, **dadurch gekennzeichnet, dass** die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt Sulfonsäure und/oder tert-Butyl.

12. Elektrolyt entsprechend einem der Ansprüche 1 bis 11, **dadurch gekennzeichnet, dass** der Elektrolyt als Lösungsmittel ein Gemisch aus Propylencarbonat und/oder Ethylencarbonat aufweist, als Leitsalz LiCl, KCl, Tetrabutylammoniumtoluolsulfonat und/oder 1-Hexyl-3-methyl-imidazolium-tris(pentafluoroethyl)-trifluorophosphat oder ein Gemisch aus zwei oder mehr dieser Komponenten aufweist und als organischen Mediator tert-butyl-Hydrochinon und/oder ein substituiertes Anthrachinon, bevorzugt Anthrachinon-2-Sulfonat aufweist.

13. Elektrolyt entsprechend einem der Ansprüche 1 bis 12, **dadurch gekennzeichnet, dass** der organische Mediator in einer Konzentration von 10⁻² mol/l oder weniger, bevorzugt von 10⁻³ mol/l oder weniger, besonders bevorzugt von 5*10⁻⁴ mol/l oder weniger, ganz besonders bevorzugt von 2*10⁻⁴ mol/l oder weniger, ganz besonders bevorzugt von 10⁻⁴ mol/l oder weniger in dem Elektrolyt enthalten ist.

14. Elektrolyt entsprechend einem der Ansprüche 1 bis 13, **dadurch gekennzeichnet, dass** der organische Mediator in einer Konzentration von 10⁻⁶ mol/l oder mehr, bevorzugt von 10⁻⁵ mol/l oder mehr, besonders bevorzugt von 5*10⁻⁵ mol/l oder mehr, ganz besonders bevorzugt von 8*10⁻⁵ mol/l oder mehr, ganz besonders bevorzugt von 10⁻⁴ mol/l oder mehr in dem Elektrolyt enthalten ist.

15. Verfahren zur Herstellung eines Elektrolyten entsprechend einem der vorhergehenden Ansprüche, umfassend die Schritte
a. Vorlegen des Lösungsmittels in einem Reaktionsgefäß
b. Zugabe des Puffers
c. Zugabe des organischen Mediators
d. Erhitzen des Gemisches unter Rühren für ca. 15 Minuten auf 150 °C
e. Rühren für etwa eine Stunde ohne weitere Hitzezufuhr bis alle Feststoffe gelöst sind
f. Abkühlen auf Raumtemperatur
g. Zugabe des Leitsalzes

## Claims

1. A liquid electrolyte for an electrochemical gas sensor, in particular for an electrochemical gas sensor which is suitable for detecting NH₃ or NH₃-containing gas mixtures, wherein the electrolyte contains at least one solvent, a conductive salt and an organic mediator, wherein the conductive salt is an ionic liquid, an inorganic salt, an organic salt or a mixture thereof, **characterised in that** the organic mediator is a polyhydroxy compound which forms a quinoid system or a naphthalene system during oxidation, wherein the organic mediator is selected from the group containing substituted ortho-dihydroxybenzenes, substituted para-dihydroxybenzenes, dihydroxynaphthalene, substituted dihydroxynaphthalane, anthrahydroquinone, substituted anthrahydroquinone.

2. An electrolyte according to claim 1, **characterised in that** the electrolyte contains a buffer, wherein the buffer is preferably a compound corresponding to
Formula I R¹-(CR²R³)ₙ-SO₃H
with n = 1, 2, 3, 4 or 5, preferably n = 2 or n = 3, wherein all R² and R³ are selected independently of one another from H, NH and OH, and wherein R¹ is selected from the group containing piperazinyl, substituted piperazinyl, N-morpholino, cycloalkyl, tris-(hydroxyalkyl)alkyl.

3. An electrolyte according to claim 2, **characterised in that** n = 2 or n = 3, wherein all R² and R³ are selected independently of one another from H, NH and OH, and wherein R¹ is selected from [4-(2-hydroxyethyl)-1]-piperazinyl, (N-morpholino), N-cyclohexyl, tris-(hydroxymethyl)methyl, wherein the buffer is preferably 3-(N-morpholino)propanesulphonic acid or 3-(N-morpholino)-ethanesulphonic acid.

4. An electrolyte according to one of claims 1 to 3, **characterised in that** the electrolyte contains as a further component a component for lowering the vapour pressure, wherein the further component is preferably an alkylene glycol or polyalkylene glycol, particularly preferably propylene glycol, ethylene glycol or a mixture of propylene glycol and ethylene glycol.

5. An electrolyte according to at least one of claims 1 to 4, **characterised in that** the solvent is selected from the group containing water and alkylene carbonate or mixtures thereof, preferably selected from the group containing water, propylene carbonate, ethylene carbonate or mixtures thereof.

6. An electrolyte according to at least one of claims 1 or 5, **characterised in that** the anion of the conductive salt is selected from the group containing halides, carbonate, sulphonate, phosphate and/or phosphonate, preferably an anion selected from the group containing alkyl sulphonate, alkenyl sulphonate, aryl sulphonate, alkyl phosphate, alkenyl phosphate, aryl phosphate, substituted alkyl sulphonate, substituted alkenyl sulphonate, substituted aryl sulphonate, substituted alkyl phosphate, substituted alkenyl phosphate, substituted aryl phosphate, halogenated phosphate, halogenated sulphonate, halogenated alkyl sulphonate, halogenated alkenyl sulphonate, halogenated aryl sulphonate, halogenated alkyl phosphate, halogenated alkenyl phosphate, halogenated aryl phosphate, particularly preferably an anion selected from the group containing fluorophosphate, alkyl fluorophosphate, aryl sulphonate,
especially preferably from the group containing perfluoroalkyl fluorophosphate, toluene sulphonate.

7. An electrolyte according to at least one of claims 1 to 6, **characterised in that** the conductive salt contains as cations metal ions, onium ions or a mixture of metal ions and onium ions.

8. An electrolyte according to claim 7, **characterised in that** the metal ions are selected from alkali metal ions or alkaline-earth metal ions, preferably from Li, K, and/or Na.

9. An electrolyte according to one of claims 7 or 8, **characterised in that** the onium ions are selected from ammonium, phosphonium, guanidinium cations and heterocyclic cations,
preferably selected from alkyl ammonium and heterocyclic cations,
particularly preferably selected from alkyl ammonium, imidazolium and/or substituted imidazolium ions, wherein the substituted imidazolium ions preferably have a structure corresponding to 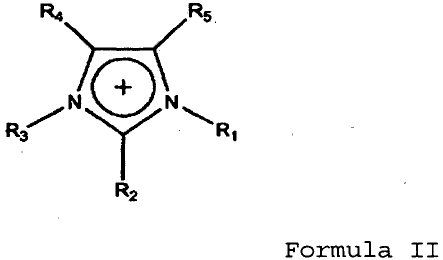 wherein R1, R2, R3, R4 and R5 can be selected independently of one another from -H, straight-chain or branched alkyl with 1 to 20 C-atoms, straight-chain or branched alkenyl with 2 to 20 C-atoms and one or more double bonds, straight-chain or branched alkinyl with 2 to 20 C-atoms and one or more triple bonds, saturated, partially or completely unsaturated cycloalkyl with 3-7 C-atoms which can be substituted with alkyl groups with 1 to 6 C-atoms, saturated partially or completely unsaturated heteroaryl, heteroaryl-C1-C6-alkyl or aryl-C1-C6-alkyl, wherein particularly preferably R2, R4 and R5 are H, and R1 and R3 are in each case independently of each other a straight-chain or branched alkyl with 1 to 20 C-atoms.

10. An electrolyte according to one of the preceding claims, **characterised in that** the organic mediator is selected from the group containing substituted 1,2- or 1,4 dihydroxybenzene, substituted hydroquinone, substituted naphtohydroquinone,
particularly preferably substituted anthrahydroquinone, substituted hydroquinone, substituted 1,2-dihydroxybenzene.

11. An electrolyte according to claim 10, **characterised in that** the substituents of the substituted anthraquinone, substituted 1,2-dihydroxybenzene and/or substituted 1,4-hydroquinone are selected from the group containing sulphonyl, tert-butyl, hydroxyl, alkyl, aryl, preferably sulphonic acid and/or tert-butyl.

12. An electrolyte according to one of claims 1 to 11, **characterised in that** the electrolyte has as a solvent a mixture of propylene carbonate and/or ethylene carbonate, has as a conductive salt LiCl, KCl, tetrabutylammonium toluene sulphonate and/or 1-hexyl-3-methyl-imidazolium-tris(pentafluoroethyl)-trifluorophospate or a mixture of two or more of these components, and has as an organic mediator tert-butyl-hydroquinone and/or a substituted anthraquinone, preferably anthraquinonone-2-sulphonate.

13. An electrolyte according to one of claims 1 to 12, **characterised in that** the organic mediator is contained in the electrolyte in a concentration of 10⁻² mol/l or less, preferably 10⁻³ mol/l or less, particularly preferably 5*10⁻⁴ mol/l or less, especially preferably 2*10⁻⁴ mol/l or less, especially preferably 10⁻⁴ mol/l or less.

14. An electrolyte according to one of claims 1 to 13, **characterised in that** the organic mediator is contained in the electrolyte in a concentration of 10⁻⁶ mol/l or more, preferably 10⁻⁵ mol/l or more, particularly preferably 5*10⁻⁵ mol/l or more, especially preferably 8*10⁻⁵ mol/l or more, especially preferably 10⁻⁴ mol/l or more.

15. A method for the production of an electrolyte according to one of the preceding claims, including the steps:
a. placing the solvent in a reaction vessel
b. addition of the buffer
c. addition of the organic mediator
d. heating the mixture whilst stirring for approximately 15 minutes to 150°C
e. stirring for about one hour without any further supply of heat until all the solids have dissolved
f. cooling to room temperature
g. addition of the conductive salt.

## Revendications

1. Électrolyte liquide pour un capteur électrochimique de gaz, en particulier pour un capteur électrochimique de gaz qui est approprié à la détection de NH₃ ou de mélanges de gaz contenant du NH₃, l'électrolyte contenant au moins un solvant, un sel conducteur et un médiateur organique, le sel conducteur étant un liquide ionique, un sel inorganique, un sel organique ou un mélange de ceux-ci, **caractérisé en ce que** le médiateur organique est un composé polyhydroxylé qui forme à l'oxydation un système quinoïde ou un système naphtalénique, le médiateur organique étant choisi dans le groupe contenant des ortho-dihydroxybenzènes substitués, des para-dihydroxy-benzènes substitués, le dihydroxynaphtalène, un dihydroxynaphtalène substitué, l'anthrahydroquinone, une anthrahydroquinone substituée.

2. Électrolyte selon la revendication 1, **caractérisé en ce que** l'électrolyte contient un tampon, le tampon étant de préférence un composé correspondant à la
formule I R¹-(CR²R³)ₙ-SO₃H
où n = 1, 2, 3, 4 ou 5, de préférence n = 2 ou n = 3, tous les radicaux R² et R³ étant choisis indépendamment les uns des autres parmi H, NH et OH, et R¹ étant choisi dans le groupe contenant les groupes pipérazinyle, pipérazinyle substitué, N-morpholino, cycloalkyle, tris(hydroxyalkyl)alkyle.

3. Électrolyte selon la revendication 2, **caractérisé en ce que** n = 2 ou n = 3, tous les radicaux R² et R³ étant choisis indépendamment les uns des autres parmi H, NH et OH, et R¹ étant choisi parmi les.groupes [4-(2-hydroxyéthyl)-1]-pipérazinyle, (N-morpholino), N-cyclohexyle, tris-(hydroxyméthyl)méthyle, le tampon étant de préférence l'acide 3-(N-morpholino)-propanesulfonique ou l'acide 3-(N-morpholino)éthane-sulfonique.

4. Électrolyte selon l'une quelconque des revendications 1 à 3, **caractérisé en ce que** l'électrolyte contient comme autre composante une composante destinée à l'abaissement de la tension de vapeur, l'autre composante étant de préférence un alkylèneglycol ou polyalkylèneglycol, de façon particulièrement préférée le propylèneglycol, l'éthylèneglycol ou un mélange de propylèneglycol et d'éthylèneglycol.

5. Électrolyte selon au moins l'une quelconque des revendications 1 à 4, **caractérisé en ce que** le solvant est choisi dans le groupe contenant l'eau et un carbonate d'alkylène ou des mélanges de ceux-ci, de préférence choisi dans le groupe contenant l'eau, le carbonate de propylène, le carbonate d'éthylène ou des mélanges de ceux-ci.

6. Électrolyte selon au moins l'une quelconque des revendications 1 et 5, **caractérisé en ce que** l'anion du sel conducteur est choisi dans le groupe contenant des halogénures, un carbonate, sulfonate, phosphate et/ou phosphonate,
de préférence un anion choisi dans le groupe contenant un alkyl-sulfonate, alcényl-sulfonate, aryl-sulfonate,
alkyl-phosphate, alcényl-phosphate, aryl-phosphate, alkyl-sulfonate substitué, alcényl-sulfonate substitué, aryl-sulfonate substitué, alkyl-phosphate substitué, alcényl-phosphate substitué, aryl-phosphate substitué, phosphate halogéné, sulfonate halogéné, alkyl-sulfonate halogéné, alcényl-sulfonate halogéné, aryl-sulfonate halogéné, alkyl-phosphate halogéné alcényl-phosphate halogéné, aryl-phosphate halogéné ;
de façon particulièrement préférée un anion choisi dans le groupe contenant un fluorophosphate, alkylfluorophosphate, aryl-sulfonate,
de façon tout particulièrement préférée dans le groupe contenant un perfluoroalkylfluorophosphate, le toluènesulfonate.

7. Électrolyte selon au moins l'une quelconque des revendications 1 à 6, **caractérisé en ce que** le sel conducteur contient comme cations des ions métalliques, des ions onium ou un mélange d'ions métalliques et d'ions onium.

8. Électrolyte selon la revendication 7, **caractérisé en ce que** les ions métalliques sont choisis parmi des ions de métaux alcalins ou des ions de métaux alcalino-terreux, de préférence parmi Li, K et/ou Na.

9. Électrolyte selon l'une quelconque des revendications 7 et 8, **caractérisé en ce que** les ions ionium sont choisis parmi les cations ammonium, phosphonium, guanidinium et des cations hétérocycliques,
de préférence choisis parmi des cations alkyl-ammonium et hétérocycliques,
de façon particulièrement préférée choisis des ions alkyl-ammonium, imidazolium et/ou imidazolium substitués, les ions imidazolium substitués présentant de préférence une structure correspondant à 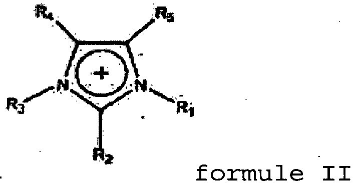 dans laquelle R1, R2, R3, R4 et R5 peuvent être choisis, indépendamment les uns des autres, parmi -H, un groupe alkyle à chaîne droite ou ramifié, ayant de 1 à 20 atomes de carbone, un groupe alcényle à chaîne droite ou ramifié, ayant de 2 à 20 atomes de carbone et comportant une ou plusieurs doubles liaisons, un groupe alcynyle à chaîne droite ou ramifié, ayant de 2 à 20 atomes de carbone et comportant une ou plusieurs triples liaisons, un groupe cycloalkyle saturé, partiellement ou totalement insaturé, ayant 3-7 atomes de carbone, qui peut être substitué par des groupes alkyle ayant de 1 à 6 atomes de carbone, un groupe hétéroaryle, hétéroaryl-alkyle(C₁-C₆) ou aryl-alkyle(C₁-C₆), saturé, partiellement ou totalement insaturé, de façon particulièrement préférée R2, R4 et R5 représentant H et R1 et R3 représentant chacun, indépendamment l'un de l'autre, un groupe alkyle à chaîne droite ou ramifié, ayant de 1 à 20 atomes de carbone.

10. Électrolyte selon l'une quelconque des revendications précédentes, **caractérisé en ce que** le médiateur organique est choisi dans le groupe contenant un 1,2- ou 1,4-dihydroxybenzène substitué, une hydroquinone substituée, une naphtohydroquinone substituée,
de façon particulièrement préférée une anthrahydroquinone substituée, une hydroquinone substituée, un 1,2-dihydroxybenzène substitué.

11. Électrolyte selon la revendication 10, **caractérisé en ce que** les substituants de l'anthraquinone substituée, du 1,2-dihydroxybenzène substitué et/ou de la 1,4-hydroquinone substituée sont choisis dans le groupe contenant des substituants sulfonyle, tert-butyle, hydroxy, alkyle, aryle, de préférence sulfonyle et/ou tert-butyle.

12. Électrolyte selon l'une quelconque des revendications 1 à 11, **caractérisé en ce que** l'électrolyte comporte comme solvant un mélange **de** carbonate de propylène et/ou carbonate d'éthylène, comme sel conducteur LiCl, KCl, le toluènesulfonate de tétrabutylammonium et/ou le tris-(pentafluoroéthyl)-trifluorophosphate de 1-hexyl-3-méthyl-imidazolium ou un mélange de deux ou plus de deux de ces composants .et comme médiateur organique la tert-butyl-hydroquinone et/ou une anthraquinone substituée, de préférence l'anthraquinone-2-sulfonate.

13. Électrolyte selon l'une quelconque des revendications 1 à 12, **caractérisé en ce que** le médiateur organique est contenu dans l'électrolyte à une concentration de 10⁻² mole/l ou moins, de préférence de 10⁻³ mole/l ou moins, de façon particulièrement préférée de 5*10⁻⁴ mole/l ou moins, de façon tout particulièrement préférée de 2*10⁻⁴ mole/l ou moins, de façon tout particulièrement préférée de 10⁻⁴ mole/l ou moins.

14. Électrolyte selon l'une quelconque des revendications 1 à 13, **caractérisé en ce que** le médiateur organique est contenu dans l'électrolyte à une concentration de 10⁻⁶ mole/l ou plus, de préférence de 10⁻⁵ mole/l ou plus, de façon particulièrement préférée de 5*10⁻⁵ mole/l ou plus, de façon tout particulièrement préférée de 8*10⁻⁵ mole/l ou plus, de façon tout particulièrement préférée de 10⁻⁴ mole/l ou plus.

15. Procédé pour la préparation d'un électrolyte selon l'une quelconque des revendications précédentes, comprenant les étapes
a. mise à disposition au préalable dû solvant dans un récipient de réaction
b. addition du tampon
c. addition du médiateur organique
d. chauffage du mélange sous agitation pendant environ 15 minutes à 150 °C
e. agitation pendant environ une heure sans autre apport de chaleur, jusqu'à ce que tous les solides soient dissous
f. refroidissement jusqu'à la température ambiante
g. addition du sel conducteur
